(19) Europäisches Patentamt
European Patent Office
Office européen des brevets

(11) Numéro de publication : **0 349 432 B1**

(12) # FASCICULE DE BREVET EUROPEEN

(45) Date de publication du fascicule du brevet : **30.09.92 Bulletin 92/40**

(51) Int. Cl.$^5$ : **C07D 207/34**

(21) Numéro de dépôt : **89401861.3**

(22) Date de dépôt : **29.06.89**

(54) **Sel de strontium, son procédé de préparation et les compositions pharmaceutiques le renfermant.**

(30) Priorité : **29.06.88 FR 8808729**

(43) Date de publication de la demande : **03.01.90 Bulletin 90/01**

(45) Mention de la délivrance du brevet : **30.09.92 Bulletin 92/40**

(84) Etats contractants désignés : **AT BE CH DE ES FR GB GR IT LI LU NL SE**

(56) Documents cités : **GB-A- 2 091 998**

(73) Titulaire : **ADIR ET COMPAGNIE**
**1 rue Carle Hébert**
**F-92415 Courbevoie Cédex (FR)**

(72) Inventeur : **Wierzbicki, Michel**
**32, rue Lucien Voilin**
**F-92800 Puteaux (FR)**
Inventeur : **Bonnet, Jacqueline**
**19, rue Charcot**
**F-75013 Paris (FR)**
Inventeur : **Tsouderos, Yannis**
**80 Elysée II**
**F-78170 La Celle-St-Cloud (FR)**

(74) Mandataire : **Reverbori, Marcelle**
**Adir et Compagnie, 1, rue Carle Hébert**
**F-92415 Courbevoie Cédex (FR)**

EP 0 349 432 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un nouveau sel de strontium, son procédé de préparation et les compositions pharmaceutiques le renfermant.

Elle concerne particulièrement le sel distrontique de l'acide (carboxyméthyl-1 carboxy-2 pyrrolyl-4)-3 pentanedioïque de formule I :

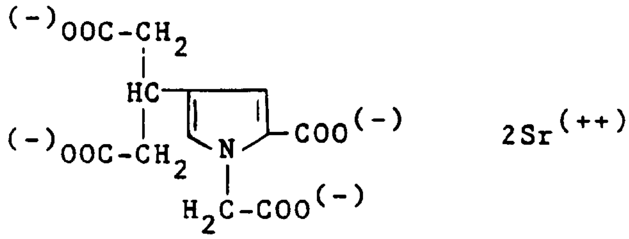

(I)

La présente invention a également pour objet le procédé de préparation du sel distrontique de formule I caractérisé en ce que :

l'on dédiazote le dérivé du thiéno [2,3-b] pyrrole de formule II :

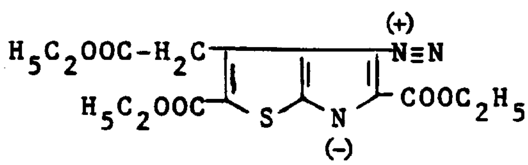

(II)

pour obtenir un composé de formule III :

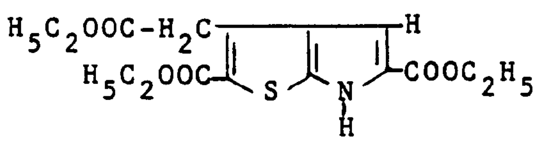

(III)

que l'on traite par le bromoacétate d'éthyle en présence de sodium dans l'éthanol anhydre pour obtenir le composé de formule IV :

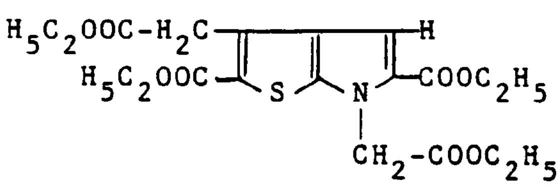

(IV)

que l'on fait réagir avec le nickel de Raney en milieu éthanol anhydre pour obtenir le composé de formule V :

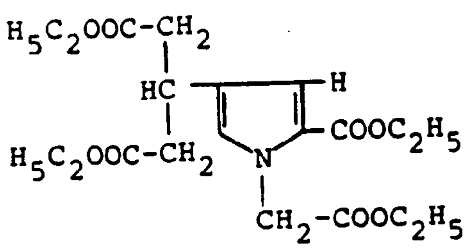

(V)

lequel est chauffé à reflux en présence de soude en milieu hydroalcoolique pour donner l'acide de for-

mule VI :

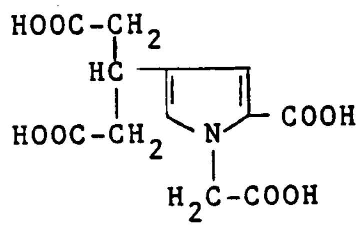

(VI)

que l'on fait réagir avec $Sr(OH)_2$ en milieu aqueux pour obtenir le sel distrontique correspondant.

Le dérivé de thiéno [2,3-b] pyrrole (II) de départ est décrit dans Bull. Soc. Chim. (1975) pages 1786-92.

L'acide (carboxyméthyl-1 carboxy-2 pyrrolyl-4)-3 pentanedioïque de formule VI est un produit nouveau qui peut être utilisé comme matière première dans l'industrie chimique et pharmaceutique, notamment dans la synthèse du sel distrontique de formule I. Il est donc à ce titre, inclus dans la présente invention.

Le sel distrontique de formule I possède des propriétés pharmacologiques et thérapeutiques intéressantes, notamment des propriétés anti-ostéoporotiques remarquables, qui, de ce fait, permettent son utilisation comme médicament notamment dans le traitement des maladies osseuses. Le sel distrontique de formule I peut également être utilisé dans le traitement du vieillissement cutané et vasculaire et des affections hépatiques.

Il est connu, de l'état antérieur de la technique que certains sels de strontium sont utilisables en thérapeutique. En particulier le brevet GB 2.091.998 revendique l'utilisation des sels de strontium avec les acides organiques usuels dans le traitement de la lithiase urinaire. Certaines publications de la littératurenotamment Gastineau, Proc. Staff. Meetings Mayo Clinic 35, 105-11 (1960) ; Skoryna, Can. Med. Assoc. 125 (7), 702-12 (1981), Skoryna, Trace Subst. Environ. Healt 18, 3-23 (1984) - font état de l'activité des lactate, gluconate et carbonate de strontium dans le traitement de l'ostéoporose.

Le sel distrontique de la présente invention, outre le fait d'être nouveau vis à vis des sels de strontium préalablement connus, présente par rapport à ces derniers, des avantages surprenants, notamment une meilleure bio-disponibilité, comme le montre l'étude pharmacologique relatée ci-après, ce qui permet d'administrer des doses chimiques moindres lors du traitement de l'ostéoporose.

La présente invention a également pour objet les compositions pharmaceutiques contenant comme principe actif le sel de strontium de formule I, mélangé ou associé à un excipient pharmaceutique approprié, comme par exemple, l'eau distillée, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent contenir de 200 à 300mg de principe actif. Elles peuvent revêtir la forme de comprimés, dragées, gélules, solutions buvables, solutions injectables ou suppositoires, et être, selon les cas, administrées par voie orale, rectale ou parentérale à la dose de 200 à 300mg 2 à 4 fois par jour.

Les exemples suivants illustrent l'invention.

**EXEMPLE 1 :**

Synthèse du sel distrontique de l'acide (carboxyméthyl-1 carboxy-2 pyrrolyl-4)-3 pentanedioïque.

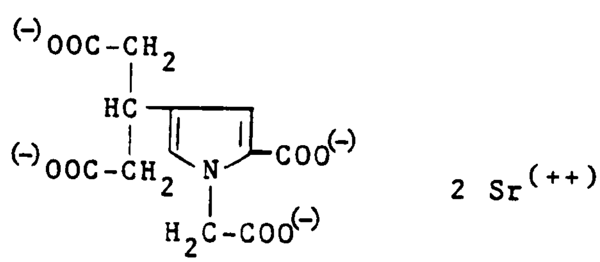

Elle comprend les étapes de préparation suivantes :

a) L'éthoxycarbonylméthyl-3 diazo-4 diéthoxycarbonyl-2,5 thiéno [2,3-b] pyrrole, {préparée selon Bull. Soc. Chim. (1975) pages 1786-92, à partir de 425g (1,03 moles) d'acétyl-6 amino-4 éthoxycarbonylméthyl-3 diéthoxycarbonyl-2,5 thiéno [2,3-b] pyrrole} additionné de quelques ml d'$H_2SO_4N$ est mis à refluer dans 5 litres d'éthanol sous courant d'azote. On distille l'acétaldéhyde formé et laisse passer un peu d'éthanol de façon à concentrer le milieu. On laisse poursuivre la réaction jusqu'à disparition totale du diazo (facile à mettre en évidence par sa coloration jaune intense - dépôt sur silice). On concentre à 2 litres d'éthanol aqueux, laisse cristalliser le produit, le filtre et le lave par deux fois 200ml d'un mélange éthanol/eau (1/3). On obtient finalement 256g d'éthoxycarbonylméthyl-3 diéthylcarbonyl-2,5 thiéno [2,3-b] pyrrole, sensiblement pur, fondant à 147-148°C. (Rendement : 70%).

b) On dissout à température ambiante 256g (0.72 moles) d'éthoxycarbonylméthyl-3 diéthoxycarbonyl-2,5 thiéno [2,3-b] pyrrole, précédemment obtenu, dans une solution d'éthylate de sodium préparée en dissolvant 33g (1,43 équivalents) de sodium dans 3 litres d'éthanol anhydre. On ajoute, en une seule fois, 240g (1,44 mole) de bromoacétate d'éthyle et on chauffe à reflux, le mélange réactionnel pendant 3 heures. On filtre le précipité formé et concentre le filtrat à environ 1,2 litre. On filtre pour isoler le nouveau précipité obtenu, réunit les deux précipités et les lave avec environ 1 litre d'eau distillée puis 500ml d'éther de pétrole. On obtient ainsi 269g de diéthoxycarbonylméthyl-3,6 diéthoxycarbonyl-2,5 thiéno [2,3-b] pyrrole, pur, P.F. : 190°C (Rendement : 85%).

c) Les 269g de produit ainsi obtenus sont mis en solution dans 8 litres d'éthanol, en tiédissant si nécessaire. On y ajoute 1600cm$^3$ de nickel de Raney et chauffe à reflux pendant une heure. On filtre ensuite l'éthanol encore tiède puis évapore l'éthanol. On obtient finalement 252g de (diéthoxycarbonyl-1,3 propyl-2)-4 éthoxycarbonyl-2 éthoxycarbonylméthyl-1 pyrrole, sensiblement pur. $Eb/_{0,02mm}$= 190°C (Rendement 100%).

d) A ces 252g de produit (0.61 mole) on ajoute 643ml de soude 4 N (2,57 moles), 210ml d'eau distillée et 210ml d'éthanol. L'ensemble est chauffé sous agitation et l'on maintient le reflux pendant 1 heure 30. On évapore ensuite l'éthanol et additionne exactement 2,57 moles d'HCl. On filtre et sèche le précipité obtenu. Le précipité sec est repris par 5 litres d'acétone contenant environ 5% d'eau. On filtre l'insoluble (Na Cl) et évapore l'acétone. Le résidu d'évaporation est dissout dans 20 litres d'eau distillée et passé sur 3,4kg de résine sulfonique Dowex HCR WE. On évapore à sec à la pompe Sihi sans chauffer. On obtient 148g d'acide (carboxyméthyl-1 carboxy-2 pyrrolyl-4)-3 pentanedioïque, sensiblement pur, sous forme de produit rose fondant à 130°C. (Rendement : 81%).

e) Les 148g (0,4946 mole) de l'acide ainsi obtenus sont additionnés de 262,9g (0,9892 mole) de $Sr(OH)_2,8H_2O$ et 5,8 litres d'eau distillée. Après dissolution à température ambiante et filtration du léger trouble on évapore à la pompe Sihi et lave le produit obtenu par 300ml d'éther éthylique anhydre. On obtient 232g de sel distrontique de l'acide (carboxyméthyl-1 carboxy-2 pyrrolyl-4)-3 pentanedioïque , dont un échantillon est recristallisé dans l'eau sous forme d'hexahydrate défini, $C_{12}H_9NO_8Sr_2$, $6H_2O$ (UV : $\lambda$ max : 265mm, $\varepsilon$ : 8500)

**EXEMPLE 2 :**

Etude pharmacologique

1) Ostéoporose expérimentale d'immobilisation chez le rat

Cette étude a montré que le sel distrontique de l'acide (carboxyméthyl-1 carboxy-2 pyrrolyl-4)-3 pentanedioïque administré quotidiennement à la dose de 940mg/kg per os chez les rats soumis à une immobilisation locale d'une patte arrière diminue la perte de substance osseuse observée chez les témoins. Ceci apparaît de façon nette et significative sur le contenu minéral osseux, sans modification significative des autres paramètres osseux ou sériques.

2) Essais de biodisponibilité chez le rat

Ces essais ont portés sur :

la biodisponibilité absolue et la bio-disponibilité relative par étude de la cinétique sérique du strontium après administration orale chez le rat du sel distrontique de l'acide (carboxyméthyl-1 carboxy-2 pyrrolyl-4)-3 pentanedioïque et des sels de strontium de comparaison , soit les chlorure et gluconate de strontium. Après administration orale unique de 67mg/kg de sel distrontique de l'acide (carboxyméthyl-1 carboxy-2 pyrrolyl-4)-3 pentanedioïque, le strontium est absorbé avec une bio-disponibilité absolue de 51%, et une concentration maximum atteinte en moins d'une heure. Le strontium passe donc rapidement et en quantité importante au niveau sérique.

La biodisponibilité du strontium apparaît comparable pour le sel distrontique de l'acide (carboxyméthyl-1 carboxy-2 pyrrolyl-4)-3 pentanedioïque et pour le chlorure de strontium, un peu supérieure pour le gluconate, si l'on compare à des doses équivalent à une même quantité de strontium élément.

Cependant, à doses de principe actif égales, le sel distrontique de l'acide (carboxyméthyl-1 carboxy-2

pyrrolyl-4)-3 pentanedioïque, comme le chlorure de strontium, en apportant une masse plus importante de strontium que ne le fait le gluconate, permet l'absorpsion d'une plus grande quantité de strontium, donc une meilleure efficacité.

## Revendications

### Revendications pour les Etats contractants suivants : AT, BE, CH, DE, FR, GB, IT, LI, LU, NL, SE

1) Le sel distrontique de l'acide (carboxyméthyl-1 carboxy-2 pyrrolyl-4)-3 pentanedioïque, de formule I :

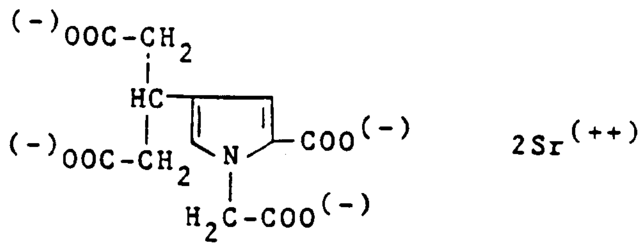

(I)

2) L'acide (carboxyméthyl-1 carboxy-2 pyrrolyl-4)-3 pentanedioïque.

3) Le procédé de préparation du sel distrontique de la revendication 1 caractérisé en ce que l'on dédiazote le dérivé du thiéno [2,3-b] pyrrole de formule II :

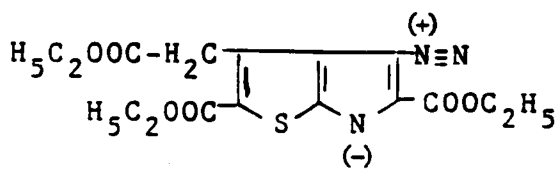

(II)

pour obtenir un composé de formule III :

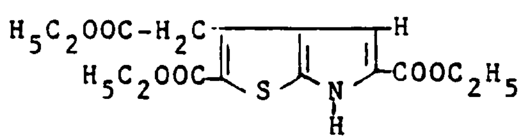

(III)

que l'on traite par le bromoacétate d'éthyle en présence de sodium dans l'éthanol anhydre pour obtenir le composé de formule IV :

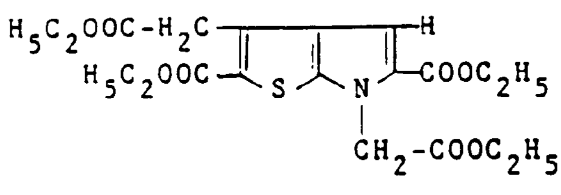

(IV)

que l'on fait réagir avec le nickel de Raney en milieu éthanol anhydre pour obtenir le composé de formule V :

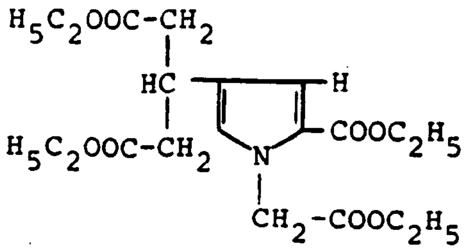

(V)

lequel est chauffé à reflux en présence de soude en milieu hydroalcoolique pour donner l'acide de formule VI :

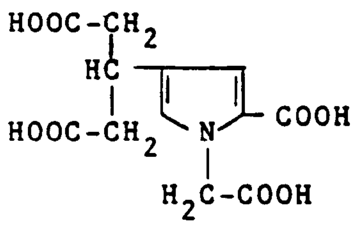

(VI)

que l'on fait réagir avec $Sr(OH)_2$ en milieu aqueux pour obtenir le sel distrontique correspondant.

4) Les compositions pharmaceutiques contenant, comme principe actif, le sel distrontique de l'acide (carboxyméthyl-1 carboxy-2 pyrrolyl-4)-3 pentanedioïque, avec des excipients pharmaceutiques appropriés.

5) Les compositions pharmaceutiques selon la revendication 4 présentées sous une forme convenant notamment pour le traitement des maladies osseuses, du vieillissement cutané et vasculaire et des affections hépatiques.

**Revendications pour les Etats contractants suivants: ES, GR**

1) Le procédé de préparation du sel distrontique de l'acide (carboxyméthyl-1 carboxy-2 pyrrolyl-4)-3 pentanedioïque, de formule I :

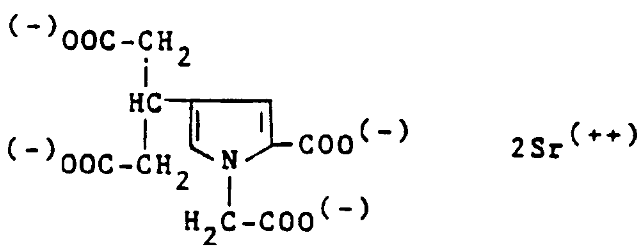

(I)

caractérisé en ce que

l'on dédiazote le dérivé du thiéno [2,3-b] pyrrole de formule II :

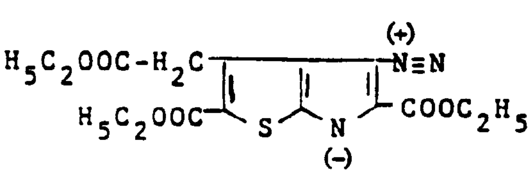

(II)

pour obtenir un composé de formule III :

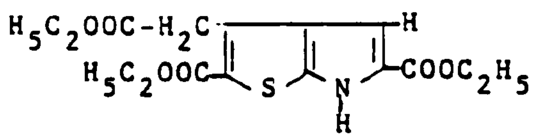

(III)

que l'on traite par le bromoacétate d'éthyle en présence de sodium dans l'éthanol anhydre pour obtenir le composé de formule IV :

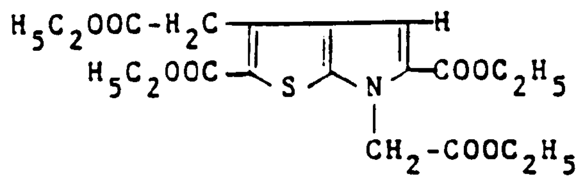

(IV)

que l'on fait réagir avec le nickel de Raney en milieu éthanol anhydre pour obtenir le composé de formule V :

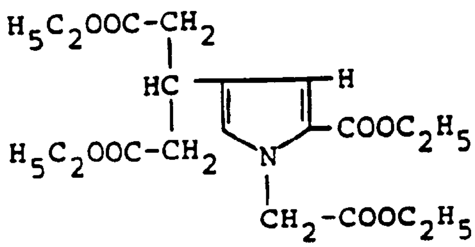

(V)

lequel est chauffé à reflux en présence de soude en milieu hydroalcoolique pour donner l'acide de formule VI :

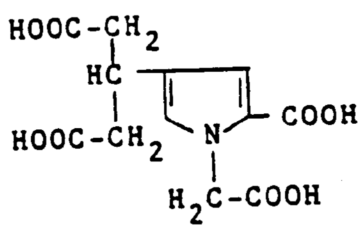

(VI)

que l'on fait réagir avec $Sr(OH)_2$ en milieu aqueux pour obtenir le sel distrontique correspondant.

2) Le procédé de préparation de l'acide (carboxyméthyl-1 carboxy-2 pyrrolyl-4)-3 pentane dioïque caractérisé en ce que l'on opère comme défini dans la revendication 1 jusqu'à l'obtention du dérivé VI.

## Claims

### Claims for the following Contracting States: AT BE CH DE FR GB IT LI LU NL SE

1. The distrontium salt of 3-(1-carboxymethyl-2-carboxy-4-pyrrolyl)-pentanedioic acid of the formula I:

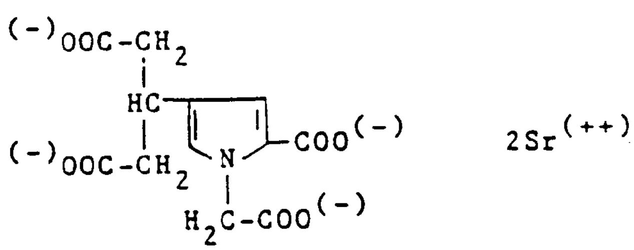

(I)

**2.** 3-(1-carboxymethyl-2-carboxy-4-pyrrolyl)-pentanedioic acid.

**3.** Process for the preparation of the distrontium salt of claim 1, characterised in that the thieno[2,3-b]pyrrole compound of the formula II:

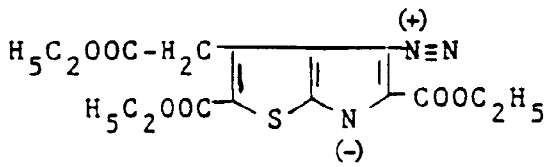

(II)

is dediazotised to obtain a compound of the formula III:

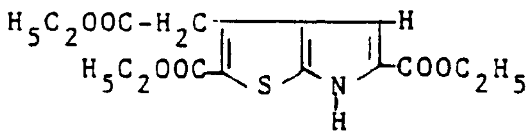

(III)

which is treated with ethyl bromoacetate in the presence of sodium in anhydrous ethanol to obtain the compound of the formula IV:

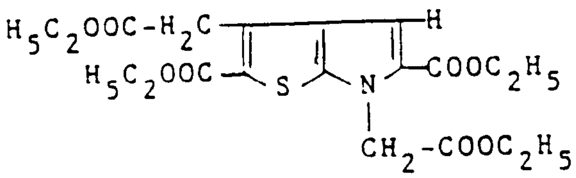

(IV)

which is reacted with Raney nickel in anhydrous ethanol medium to obtain the compound of the formula V:

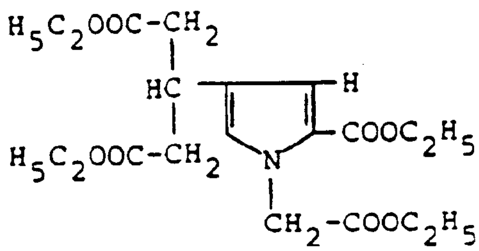

(V)

which is heated under reflux in the presence of sodium hydroxide in aqueous alcoholic medium to give the acid of the formula VI:

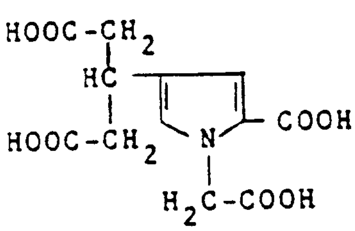

(VI)

which is reacted with $Sr(OH)_2$ in aqueous medium to obtain the corresponding distrontium salt.

**4.** Pharmaceutical compositions containing as active ingredient the distrontium salt of 3-(1-carboxymethyl-2-carboxy-4-pyrrolyl)-pentanedioic acid, with suitable pharmaceutical excipients.

5. Pharmaceutical compositions according to claim 4, presented in a form suitable especially for the treatment of bone diseases, ageing of the skin and vascular ageing and hepatic disorders.

**Claims for the following Contracting States: ES GR**

1. Process for the preparation of the distrontium salt of 3-(1-carboxymethyl-2-carboxy-4-pyrrolyl)-pentanedioic acid of the formula I:

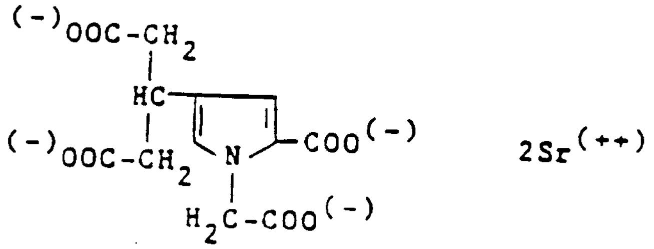

(I)

characterised in that
the thieno[2,3-b]pyrrole compound of the formula II:

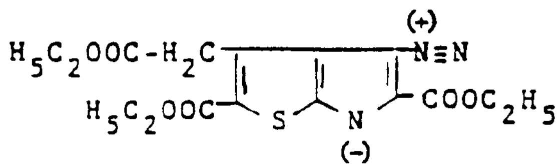

(II)

is dediazotised to obtain a compound of the formula III:

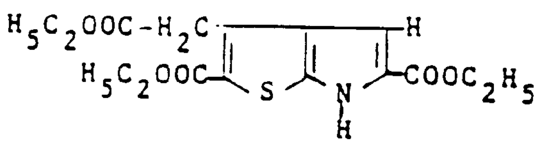

(III)

which is treated with ethyl bromoacetate in the presence of sodium in anhydrous ethanol to obtain the compound of the formula IV:

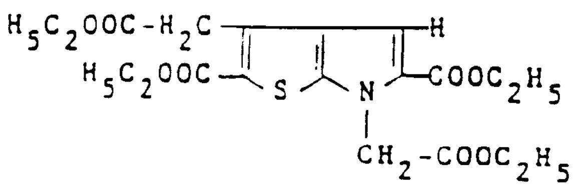

(IV)

which is reacted with Raney nickel in anhydrous ethanol medium to obtain the compound of the formula V:

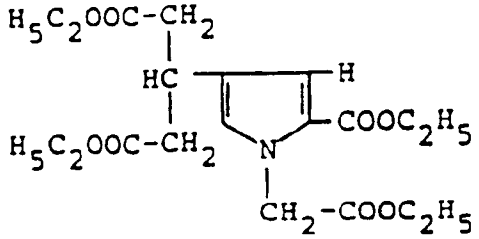

(V)

which is heated under reflux in the presence of sodium hydroxide in aqueous alcoholic medium to give

the acid of the formula VI:

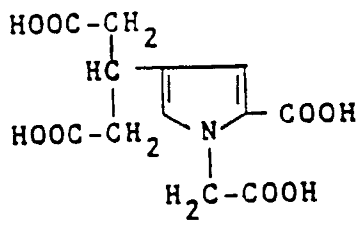

(VI)

which is reacted with $Sr(OH)_2$ in aqueous medium to obtain the corresponding distrontium salt.

2. Process for the preparation of 3-(1-carboxymethyl-2-carboxy-4-pyrrolyl)-pentanedioic acid, characterised in that the procedure defined in claim 1 is carried out until the compound VI is obtained.

**Patentansprüche**

**Patentansprüche für folgende Vertragsstaaten: AT BE CH DE FR GB IT LI LU NL SE**

1. Distrontiumsalz der 3-(1-Carboxymethyl-2-carboxy-pyrrol-4-yl)-pentandisäure der Formel I:

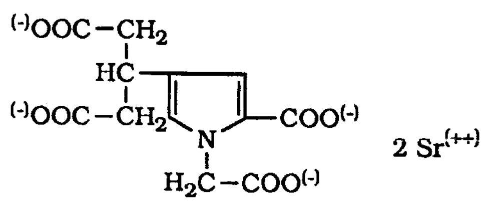

(I)

2. 3-(1-Carboxymethyl-2-carboxy-pyrrol-4-yl)-pentandisäure.

3. Verfahren zur Herstellung des Distrontiumsalzes nach Anspruch 1, **dadurch gekennzeichnet**, daß man das Thieno[2,3-b]pyrrol-Derivat der Formel II:

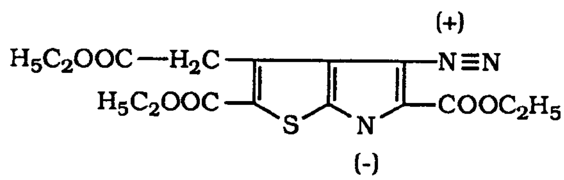

(II)

dediazotiert unter Bildung einer Verbindung der Formel III:

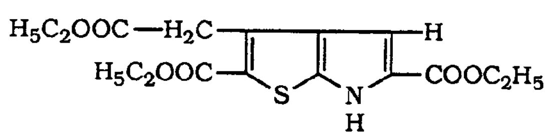

(III)

die man mit Bromessigsäureethylester in Gegenwart von Natrium in wasserfreiem Ethanol behandelt zur Bildung der Verbindung der Formel IV:

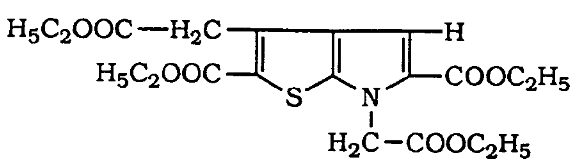

(IV)

die man mit Raney-Nickel in wasserfreiem ethanolschem Medium zur Bildung der Verbindung der Formel V:

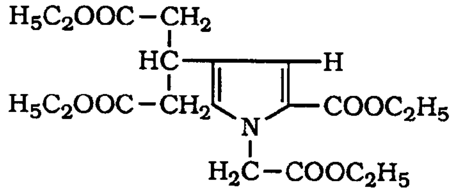

(V)

umsetzt, welche man in Gegenwart von Natriumhydroxid in wäßrig-alkoholischem Medium zum Sieden am Rückfluß erhitzt zur Bildung der Säure der Formel VI:

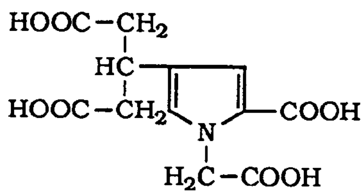

(VI)

welche man in wäßrigen Medium mit $Sr(OH)_2$ umsetzt zur Bildung des entsprechenden Distrontiumsalzes.

**4.** Pharmazeutische Zubereitungen enthaltend als Wirkstoff das Distrontiumsalz der 3-(1-Carboxymethyl-2-carboxy-pyrrol-4-yl)-pentandisäure mit geeigneten pharmazeutischen Trägermaterialien.

**5.** Pharmazeutische Zubereitungen nach Anspruch 4 in einer Form, die insbesondere in einer zur Behandlung von Knochenerkrankungen, des Alterns der Haut und der Gefäße und von Leberstörungen geeigneten Form vorliegen.

**Patentansprüche für folgende Vertragsstaaten: ES GR**

**1.** Verfahren zur Herstellung des Distrontiumsalzes der 3-(1-Carboxymethyl-2-carboxy-pyrrol-4-yl) -pentandisäure der Formel I:

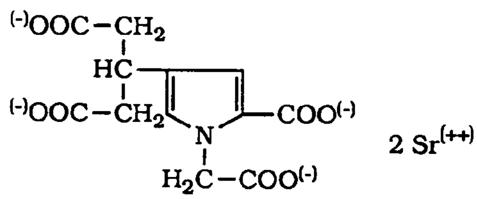

(I)

**dadurch gekennzeichnet**, daß man das Thieno[2,3-b]pyrrol-Derivat der Formel II:

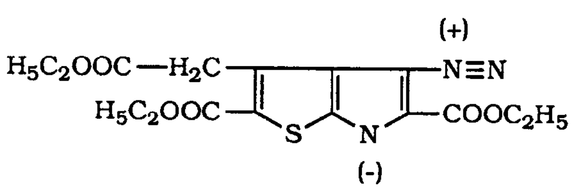

(II)

dediazotiert unter Bildung einer Verbindung der Formel III:

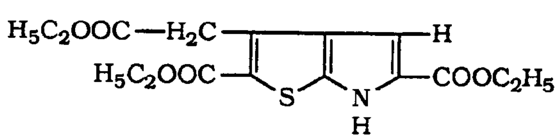

(III)

die man mit Bromessigsäureethylester in Gegenwart von Natrium in wasserfreiem Ethanol behandelt zur Bildung der Verbindung der Formel IV:

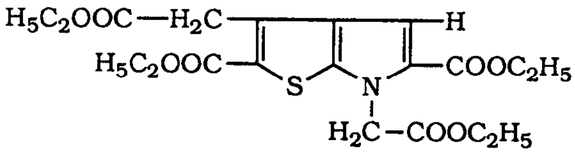

(IV)

die man mit Raney-Nickel in wasserfreiem ethanolischem Medium zur Bildung der Verbindung der Formel V:

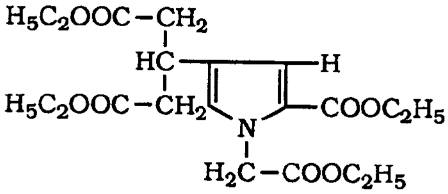

(V)

umsetzt, welche man in Gegenwart von Natriumhydroxid in wäßrig-alkoholischem Medium zum Sieden am Rückfluß erhitzt zur Bildung der Säure der Formel VI:

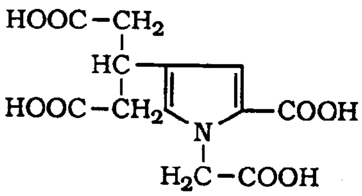

(VI)

welche man in wäßrigen Medium mit $Sr(OH)_2$ umsetzt zur Bildung des entsprechenden Distrontiumsalzes.

2. Verfahren zur Herstellung der 3-(1-Carboxymethyl-2-carboxy-pyrrol-4-yl)-pentandisäure, **dadurch gekennzeichnet**, daß man nach der Verfahrensweise des Anspruchs 1 bis zum Erhalt des Derivats der Formel VI vorgeht.